# EUROPEAN PATENT APPLICATION

(11) **EP 3 502 056 A1**
(43) Date of publication of application: **26.06.2019**
(21) Application number: 17306919.6
(22) Date of filing: 22.12.2017
(51) Int. Cl.: C01B 33/20, C01B 33/22, C01B 33/40, C01B 33/42

(54) **CO-SYNTHESIS OF PHYLLOMINERALS WITH METALLIC PARTICLES AND PRODUCTS OBTAINED THEREFROM**

(71) Applicant: IMERTECH SAS, 75015 Paris (FR); Centre National de la Recherche Scientifique (C.N.R.S.), 75794 Paris Cedex 16 (FR); Université Paul Sabatier Toulouse III, 31400 Toulouse (FR)
(72) Inventor: Le Roux, Christophe, 31290 Avignonet Lauragais (FR); Martin, François, 31570 Sainte-Foy-d'Aigrefeuille (FR); Micoud, Pierre, 31390 Peyssies (FR); Féry-Forgues, Suzanne, 82300 Monteils (FR); Poirier, Mathilde, 31400 Toulouse (FR); Aymonier, Cyril, 33130 Bègles (FR); Carême, Christel, 31850 Mondouzil (FR); Claverie, Marie, 40150 Hassegor (FR)
(74) Representative: Haseltine Lake Kempner LLP

(57) **Abstract**

The present invention relates to methods for producing mixtures comprising noble metal and phyllomineral, and compositions obtained from said methods.

## Description

### FIELD OF THE INVENTION

The present invention relates to methods for producing particulate mixtures comprising particles of at least one phyllomineral and of at least one noble metal, wherein the said noble metal particles are intimately linked to the said phyllomineral particles. The invention also relates to the products obtained from the said methods and their uses.

### BACKGROUND OF THE INVENTION

Phyllominerals ("sheet minerals") are minerals having a crystalline structure comprising at least one tetrahedral layer and at least one octahedral layer. Phyllosilicates (or "sheet silicates") are natural phyllominerals constituted by stacks of parallel sheets made up of tetrahedral and octahedral layers, and forming parallel silicate sheets of silicate tetrahedra. Examples of natural phyllosilicates are talc, mica, chlorite, kaolinite, among many others. Phyllosilicates may also be prepared artificially. As used herein, the term "phyllomineral" specifically incorporates natural phyllosilicates and synthetic phyllosilicates, including talc, mica, chlorite, kaolinite or the like.

Phyllominerals and phyllosilicates have many industrial applications. In fine particulate form they may be used, for example, as fillers in thermoplastics, elastomers, paper, paint, lacquer, textiles, metallurgy, pharmaceuticals, cosmetics, phytosanitary preparations, plant protection products or fertilisers, in which the phyllominerals or phyllosilicates are used as inert fillers or functional fillers.

WO 2013/004979 A1 discloses a synthetic procedure for producing phyllominerals and phyllosilicates such as talc in a batch process. WO 2015/159006 A1 discloses continuous processes for producing phyllominerals and phyllosilicates such as talc, mica, or montmorillonite.

A common problem of all the prior art processes for artificially producing phyllominerals or phyllosilicates is the tendency of the obtained particulates to agglomerate or cake, leading to difficulties when applying the said synthetic particles, for example when putting them into suspension. The state of the art therefore constitutes a problem.

### SHORT DESCRIPTION OF THE INVENTION

The present invention is defined in the appended claims.

In particular, the present invention is embodied by a method comprising the steps of providing a phyllomineral source, providing a noble metal source, combining the previously provided phyllomineral source with the previously provided noble metal source, and optionally hydrothermally treating the obtained mixture. It was found that with the said method, particulate compositions may be obtained wherein noble metal nanoparticles are intimately bound to the surface of phyllomineral particles, in particular on the edge surfaces of the phyllomineral particles.

According to one embodiment of the present invention, the said method may employ phyllomineral precursors or actual phyllominerals as the phyllomineral source, or mixtures of phyllomineral precursors and actual phyllominerals. According to one embodiment of the present invention, the said phyllomineral is a non-swelling phyllomineral. Similarly, according to one further embodiment of the present invention, the said method may employ noble metal precursors such as noble metal salts, or actual noble metals in their metallic state as the noble metal source, or mixtures of noble metal precursors and actual noble metals in their metallic state. It was found that the method according to the invention was sufficiently versatile that the said phyllomineral and/or the said noble metal forming part of the product may either be formed *in situ* from suitable precursors, or they may already be provided in their desired form as phyllomineral and/or noble metal in its metallic state respectively. This versatility gives the user good flexibility depending on availability of raw materials and laboratory equipment.

According to one further embodiment of the present invention, the said phyllomineral may be a single phyllomineral or a mixture of various phyllominerals. For example the phyllomineral source may be a talc or a talc source, or a mica or a mica source etc., or a mixture of talc and mica, or a mixture of talc source and mica source, etc. Similarly, according to one further embodiment of the present invention, the said noble metal may be a single noble metal or a mixture of noble metals. For example the noble metal source may be metallic gold or a gold salt, or metallic platinum or a platinum salt etc., or it may be a mixture of metallic gold and metallic platinum, or a mixture of a gold salt and a platinum salt, or even a mixture of metallic gold and a platinum salt, etc.

According to one embodiment of the present invention, the said phyllomineral may be selected from talc, mica, kaolinite, chlorite or mixtures thereof. According to a further embodiment of the present invention the said noble metal may be selected from gold, platinum, palladium, ruthenium, rhodium, iridium, osmium, silver, or mixtures thereof.

According to particular embodiments, the method of the invention may entail the use of talc precursors and/or a gold salt. According to one of these embodiments, the said phyllomineral source may be a talc precursor in the form of (i) an aqueous solution comprising a soluble metasilicate and/or metagermanate, optionally in combination with a carboxylate, and (ii) an aqueous solution comprising a soluble metal salt. According to a further one of these embodiments the noble metal source may be a precursor to metallic gold in the form of an aqueous solution comprising a soluble gold salt. It was found that such combinations were particularly well suited for the present invention.

According to one of the embodiments mentioned above, the soluble metasilicate and/or the soluble metagermanate may be selected from the respective sodium or potassium salts, or mixtures thereof. According to one further of the embodiments mentioned above, the optional carboxylate is a metal carboxylate, the metal being selected from sodium or potassium, or mixtures thereof. This carboxylate may be selected from formate, acetate, propionate, butyrate, isobutyrate, valerate, isovalerate or mixtures thereof. Furthermore, the metal in the soluble metal salt of (ii) in the phyllomineral source may be a divalent metal selected from magnesium, cobalt, zinc, copper, manganese, iron, nickel, chromium or mixtures thereof. The metal salt may be selected from those metal salts that are able to react with metasilicate or metagermanate in order to form a synthetic phyllomineral precursor. In particulate, the metal salt may be selected from metal salts soluble in a liquid medium, such as water, for example dicarboxylate salts, sulfates, nitrates, chlorides, and the like.

According to further embodiments, the noble metal in the soluble noble metal salt may be selected from gold, palladium, platinum, iridium, rhodium, ruthenium, osmium, silver or mixtures thereof. It was found that these were particularly suitable conditions of the inventive method.

According to a further embodiment of the present invention, the step of combining the previously provided phyllomineral source with the previously provided noble metal source may be carried out by mixing the said noble metal source with the said components of the phyllomineral source in any order desired. For example, the said components of the phyllomineral source may be mixed first and the said noble metal source added afterwards. For example, the said component (i) of the phyllomineral source may be mixed with the said noble metal source first and the said component (ii) of the phyllomineral source added afterwards. For example, the said component (ii) of the phyllomineral source may be mixed with the said noble metal source first and the said component (i) of the phyllomineral source added afterwards. For example, the said components of the phyllomineral source and the said noble metal source may be mixed simultaneously. It was found that simple mixing was sufficient for the method according to the invention.

According to a further embodiment of the present invention, the optional hydrothermal treatment, if carried out, may be carried out by heating at a temperature between 100°C and 600°C for a duration of 10 minutes to 30 days. According to one embodiment, the temperature of the heat treatment may be between 150°C and 400°C, or between 150°C and 370°C, or between 200°C and 350°C. According to one embodiment, the duration of the heat treatment may be from 30 minutes to 15 days, or from 1 hour to 24 hours, or from 2 hours to 12 hours. According to one embodiment, this heating may be achieved by microwave heating, by convection heating, or combinations thereof. It was found that the optional hydrothermal treatment may or may not be required in order to achieve the desired products through the method of the invention. Depending on the reactants selected, hydrothermal treatment may be required to be more or less stringent, by adapting temperature and duration in accordance with the knowledge of the skilled person in the art.

According to some embodiments, the said optional hydrothermal treatment may occur as a batch process in an autoclave under autogeneous pressure, or as a continuous process under a pressure of 1 MPa or more, as shown in WO 2015/159006 A1.

According to one embodiment of the present invention, a further step may be included prior to hydrothermal treatment, which is the addition of carboxylate salts R-COOM' as disclosed in WO 2012/085239 A1, wherein M' is either Na or K, and R is either H or alkyle having less than 10 carbon atoms.

According to a further embodiment of the present invention, the product obtained after the combination step or after hydrothermal treatment step, if carried out, may be dried, washed and/or centrifuged in order to obtain a final product with adequate storage, transport and durability properties. In accordance with the present invention, it was found that the particulate products obtained have a reduced tendency to agglomerate or cake. This leads to more flexible drying, washing and/or centrifugation requirements, since according to the state of the art, the particulate products often agglomerate or stick together at this stage.

Also part of the present invention are the particulate compositions obtained by the method of the invention. The obtained particulate compositions comprise a combination of phyllomineral with noble metal, wherein the noble metal is in the form of nanoparticles intimately connected to the surface of the phyllosilicate particles. In the particulate compositions according to the present invention, the said noble metal nanoparticles are probably preferably connected on the edges of the phyllomineral platelets, i.e. on the narrow ends of the platelet particles, rather than on the large flat faces.

In accordance with some embodiments of the present invention, and depending on the choice of phyllomineral(s) and noble metal(s) employed in the method according to the invention, the particulate compositions may comprise, as the phyllomineral, talc, mica, kaolinite, chlorite or mixtures thereof, and as the noble metal gold, iridium, platinum, palladium, ruthenium, rhodium, osmium, silver, or mixtures thereof. For example, according to some embodiments of the invention, the noble metal may be a mixture of gold and platinum, or a mixture of gold and palladium, or a mixture of rhodium and platinum and palladium. In one particular embodiment of the present invention, the particulate composition comprises talc particles with gold nanoparticles connected to the talc platelet surfaces on or in the vicinity of the platelet edges.

In accordance with some embodiments of the present invention, the particulate composition comprises phyllomineral platelets having a diameter along their longest axis of 10 nm to 1 mm, as measured in accordance with field emission gun scanning electron microscopy (FEG-SEM) or transmission electron microscopy (TEM). For example, the phyllomineral platelets may have a diameter along their longest axis of 25 nm to 1 mm, or of 50 nm to 10000 nm, for example from 100 nm to 1000 nm or from 200 nm to 750 nm, such as for example about 100 nm, or about 150 nm, or about 200 nm, or about 250 nm, or about 300 nm, or about 350 nm, or about 400 nm, or about 450 nm, or about 500 nm, or about 550 nm, or about 600 nm, or about 650 nm, or about 700 nm, or about 750 nm, or about 800 nm, or about 850 nm, or about 900 nm, or about 950 nm, or about 1000 nm.

In accordance with some embodiments of the present invention, the particulate composition comprises noble metal particles having an average particle diameter in the range of 1 nm to 100 nm, as measured in accordance with FEG-SEM or TEM. For example, the noble metal particles may have an average particle diameter of 2 nm to 50 nm, or of 3 nm to 30 nm, for example from 4 nm to 20 nm or from 5 nm to 15 nm, such as for example about 5 nm, or about 6 nm, or about 7.5 nm, or about 10 nm, or about 12.5 nm, or about 15 nm, or about 20 nm.

Finally, in accordance with further embodiments of the present invention, the methods of the invention and the particulate compositions of the present invention may be used, for example, in the production of catalysts, cosmetics, pharmaceuticals or fillers for polymers, papers, textiles, ceramics, coatings, paints, or food additives. For example, the particulate compositions of the present invention may be used as IR-blockers in cosmetics.

### SHORT DESCRIPTION OF THE FIGURES

The invention will be further illustrated by reference to the following figures:
Fig. 1 represents a field emission gun scanning electron microscopy (FEG-SEM) image of a synthetic talc (dark gray) comprising 10,000 ppm Au (white spots);
Fig. 2a represents a transmission electron microscopy (TEM) image of the surface of a synthetic talc (bright) comprising 10,000 ppm Au (black);
Fig. 2b represents the same TEM image as Fig. 2a with a larger scale.

It is understood that the following description and references to the figures concern exemplary embodiments of the present invention and shall not be limiting the scope of the claims.

### DETAILED DESCRIPTION OF THE INVENTION

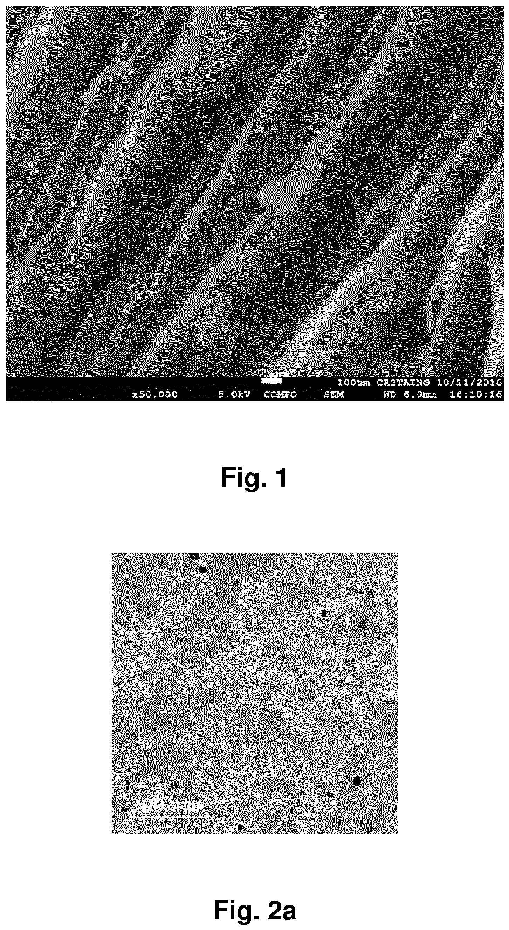

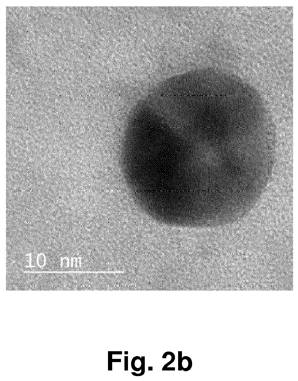

The present invention according to the appended claims provides for methods to produce novel particulate compositions comprising a particulate phyllomineral having noble metal nanoparticles intimately connected to the surfaces of the phyllomineral platelet particles. The exact nature of the connection between the noble metal and the phyllomineral is not known, but the relative arrangement of phyllominerals and noble metals may merely be observed by electron microscopy, as illustrated in Fig. 1 (FEG-SEM), and Figs. 2a and 2b (TEM).

As used herein, FEG-SEM and TEM have been used to determine the sizes and dimensions according to methods known to the skilled person in the art, and as shown, for example in WO 2015/159006 A1 WO 2013/093376 A1.

Although not wishing to be bound by theory, it is believed that the noble metal nanoparticles preferably connect to the -(Si)OH-ends and -(Mg)OH-ends of the platelets.

In accordance with the present invention, the compositions comprise a larger proportion of phyllomineral and a lower proportion of noble metal. For example, the weight ratio of phyllomineral to noble metal may be in the range of 1:1 to 10⁶:1. As used herein, the amount of noble metal in the compositions in accordance with the invention is expressed as parts per million (ppm), wherein the ppm indicates the weight ratio of noble metal to phyllomineral.

The versatility of the methods of the present invention lies in that, on the one hand, the phyllomineral source may either be a phyllomineral or a phyllomineral precursor, such as e.g. an aqueous solution comprising a soluble metasilicate and/or metagermanate, optionally in combination with a carboxylate, and the noble metal source may either be a noble metal or a noble metal salt.

For example, the method may be carried out by combining a phyllomineral such as a particulate talc with a noble metal, such as gold nanoparticles.

For example, the method may be carried out by combining a phyllomineral precursor, such as an aqueous solution comprising a soluble metasilicate and/or metagermanate, optionally in combination with a carboxylate with a noble metal, such as gold nanoparticles.

For example, the method may be carried out by combining a phyllomineral such as a particulate talc with a noble metal precursor, such as a soluble gold salt.

For example, the method may be carried out by combining a phyllomineral precursor, such as an aqueous solution comprising a soluble metasilicate and/or metagermanate, optionally in combination with a carboxylate with a noble metal precursor, such as a soluble gold salt.

Furthermore, the versatility is further determined by the fact that the order of addition of the various components does not considerably affect the reaction and reaction products. Why the exact nature of the final product obtained may incur slight changes on the basis of the order of addition of components during the formation, all combinations lead to the products of the invention being formed.

It should be noted that the present invention may comprise any combination of the features and/or limitations referred to herein, except for combinations of such features which are mutually exclusive. The foregoing description is directed to particular embodiments of the present invention for the purpose of illustrating it. It will be apparent, however, to one skilled in the art, that many modifications and variations to the embodiments described herein are possible. All such modifications and variations are intended to be within the scope of the present invention, as defined in the appended claims.

### EXAMPLES

### Example 1

This Example uses an aqueous synthetic talc dispersion as a phyllomineral source and gold nanoparticles as a noble metal source.

In a beaker, 6.3 mL of a gold nanoparticles solution (0.05 mg/mL) was added to 32.6 g aqueous synthetic talc dispersion (corresponding to 1.5 g of dry talc). The obtained mixture was diluted with 150 mL of distilled water, mixed and sonicated for several minutes. Once well dispersed, the suspension was centrifuged at 9000 rpm during 1h30. At the end, the supernatant was perfectly clear and colourless and the residue of synthetic talc was reddish, indicating the presence of gold nanoparticles.

The theoretical loading comes to 0.315 mg gold for 1.5 g talc, corresponding to a loading of 210 ppm. Attempts to run the experiment at a theoretical loading of 3000 ppm or higher led to a red coloration of the supernatant after centrifugation, indicating that not all gold nanoparticles integrated into the talc.

### Example 2

This Example uses hydrated sodium metasilicate and magnesium acetate as a phyllomineral source forming talc in situ, and chlorauric acid as a noble metal source, forming gold in situ.

42.4 g (0.2 mol) sodium metasilicate pentahydrate was dissolved in 300 mL distilled water in a first beaker (A) under magnetic stirring and ultrasound. 103.5 g anhydrous sodium acetate was added. In a second beaker (B), 32.17 g (0.15 mol) magnesium acetate tetrahydrate was dissolved in 100 mL acetic acid 1M under magnetic stirring and ultrasound. 0.8 mL of a 10 mg/mL HAuCl₄ trihydrate-solution was added to beaker (B). The content of beaker (B) was rapidly added to the content of beaker (A) with manual stirring to obtain a white suspension. The obtained aqueous suspension was treated in a hydrothermal reactor for 6 h at 300°C under autogeneous pressure (85 bar). At the end of the hydrothermal treatment, a red gel was obtained, which was washed with distilled water several times. A red paste was obtained which may be dried in a desiccator, an oven (120°C) or a centrifuge to obtain a violet particulate solid.

The theoretical loading comes to 4 mg gold for 18.96 g talc, corresponding to a loading of about 210 ppm. The experiment was repeated for theoretical loadings of 100 ppm, 500 ppm and 10000 ppm, each time giving a clear and colourless supernatant after centrifugation, indicating that all gold nanoparticles formed in situ had linked to the talc formed in situ.

### Example 3

This Example uses hydrated sodium metasilicate and magnesium acetate as a phyllomineral source forming talc in situ, and a mixture of chlorauric acid and chloroplatinic acid as a noble metal source, forming gold and platinum in situ.

42.4 g (0.2 mol) sodium metasilicate pentahydrate was dissolved in 300 mL distilled water in a first beaker (A) under magnetic stirring and ultrasound. 103.5 g anhydrous sodium acetate was added. In a second beaker (B), 32.17 g (0.15 mol) magnesium acetate tetrahydrate was dissolved in 100 mL acetic acid 1M under magnetic stirring and ultrasound. 1.90 mL of a 10 mg/mL HAuCl₄ trihydrate-solution and 2.45 mL of a 10.25 mg/mL H₂PtCl₆ hexahydrate solution were added to beaker (B). The content of beaker (B) was rapidly added to the content of beaker (A) with manual stirring to obtain a white suspension. The obtained aqueous suspension was treated in a hydrothermal reactor for 6 h at 300°C under autogeneous pressure (85 bar). At the end of the hydrothermal treatment, a grey gel was obtained, which was washed with distilled water several times.

The theoretical loading comes to 9.48 mg gold and 9.48 mg platinum for 18.96 g talc, corresponding to a loading of about 500 ppm for each of gold and platinum.

### Example 4

This Example uses hydrated sodium metasilicate and magnesium acetate as a phyllomineral source forming talc in situ, and a mixture of chloroplatinic acid, sodium tetrachloropalladate(II) and ammonium hexachlororhodate(III) as a noble metal source, forming platinum, palladium and rhodium in situ.

42.4 g (0.2 mol) sodium metasilicate pentahydrate was dissolved in 300 mL distilled water in a first beaker (A) under magnetic stirring and ultrasound. 103.5 g anhydrous sodium acetate was added. In a second beaker (B), 32.17 g (0.15 mol) magnesium acetate tetrahydrate was dissolved in 100 mL acetic acid 1M under magnetic stirring and ultrasound. 4.90 mL of a 10.25 mg/mL H2PtCI6 hexahydrate solution, 5.23 mL of a 10 mg/mL Cl₄Na₂Pd solution and 6.55 mL of a 10.39 mg/mL RhCl₆(NH₄)₃ solution were added to beaker (B). The content of beaker (B) was rapidly added to the content of beaker (A) with manual stirring to obtain a white suspension. The obtained aqueous suspension was treated in a hydrothermal reactor for 6 h at 300°C under autogeneous pressure (85 bar). At the end of the hydrothermal treatment, a dark gel was obtained, which was washed with distilled water several times.

The theoretical loading comes to 18.96 mg platinum, 18.96 mg palladium and 18.96 mg rhodium for 18.96 g talc, corresponding to a loading of about 1000 ppm for each of platinum, palladium and rhodium.

### Example 5

This Example uses hydrated sodium metasilicate and magnesium acetate as a phyllomineral source forming talc in situ, and gold nanoparticles as a noble metal source.

42.4 g (0.2 mol) sodium metasilicate pentahydrate was dissolved in 300 mL distilled water in a first beaker (A) under magnetic stirring and ultrasound. 103.5 g anhydrous sodium acetate was added. In a second beaker (B), 32.17 g (0.15 mol) magnesium acetate tetrahydrate was dissolved in 100 mL acetic acid 1M under magnetic stirring and ultrasound. 80 mL of a gold nanoparticles solution (0.05 mg/mL) was added to beaker (B). During the mixture, the red solution of gold nanoparticles turns instantaneously to a deep blue/dark solution. The content of beaker (B) was rapidly added to the content of beaker (A) with manual stirring to obtain a white suspension. The obtained aqueous suspension was treated in a hydrothermal reactor for 6h at 300°C under autogenous pressure. At the end of the hydrothermal treatment, a red gel was obtained, which was washed with distilled water several times and then centrifuged to obtain a blue-violet product and a clear and colourless supernatant.

The theoretical loading comes to 4 mg gold for 18.96 g talc, corresponding to a loading of about 210 ppm.

### Example 6

This Example uses an aqueous synthetic talc dispersion as a phyllomineral source and chlorauric acid as a noble metal source, forming gold in situ.

In a beaker, 43.48 g aqueous synthetic talc dispersion (corresponding to 2 g dry talc) was added to 84 µL of a HAuCl₄ trihydrate solution (10 mg/mL). Distilled water was added up to 150 mL. The suspension was then mixed under magnetic stirring and sonication. Once well dispersed, the mixture was placed in an autoclave at 300°C and 86 bars during 1 hour. At the end of the hydrothermal treatment, a red paste was obtained and centrifuged to recover the product.

The theoretical loading comes to 4 mg gold for 18.96 g talc, corresponding to a loading of about 210 ppm.

### Example 7

This example uses natural talc as a phyllomineral source and chlorauric acid as a noble metal source, forming gold in situ.

In a beaker, 2 g dry natural talc (white powder) was added to 4 mL of a HAuCl₄ trihydrate solution (10 mg/mL). Distilled water was added up to 20 mL. The suspension was mixed under magnetic stirring and sonication. The mixture was then placed in an autoclave at 300°C and 86 bars during 6 hours. At the end of the hydrothermal treatment, a red suspension was obtained and placed in an oven at 120°C to dry the product.

The theoretical loading comes to 20 mg gold for 2 g talc, corresponding to a loading of about 10000 ppm.

### Example 8:

This example uses natural talc as a phyllomineral source and chlorauric acid as a noble metal source, forming gold in situ.

The preparation is the same that the example 2. The aqueous suspension obtained after mixing the phyllomineral and gold sources is not hydrothermally treated, but kept at room temperature. The suspension becomes reddish as soon as the day after and becomes more and more reddish with time, indicating that there is reduction of gold and synthetization of gold particles.

## Claims

1. Method of preparation of a mixture comprising noble metal and phyllomineral, the method comprising the steps of
(a) provision of a phyllomineral source;
(b) provision of a noble metal source;
(c) combination of the phyllomineral source of step (a) with the noble metal source of step (b); and
(d) optionally hydrothermal treatment of the mixture obtained at the end of step (c).

2. Method according to claim 1, wherein the phyllomineral is a non-swelling phyllomineral.

3. Method according to claim 1 or 2, wherein the said phyllomineral source is selected from the group consisting of a phyllomineral precursor, a phyllomineral and mixtures thereof, and wherein the said noble metal source is selected from the group consisting of a noble metal compound, a noble metal in its metallic state and mixtures thereof.

4. Method according to any one of the previous claims, wherein the phyllomineral of the said phyllomineral source is a single phyllomineral or a mixture of phyllominerals, and wherein the noble metal of the said noble metal source is a single noble metal or a mixture of noble metals.

5. Method according to any one of the previous claims, wherein the phyllomineral of the said phyllomineral source is selected from the group consisting of talc, mica, chlorite, kaolinite and mixtures thereof, and wherein the noble metal of the said noble metal source is selected from the group consisting of gold, palladium, platinum, iridium, ruthenium, rhodium, or mixtures thereof.

6. Method according to any one of the previous claims, wherein the said phyllomineral source is a combination of (i) an aqueous solution comprising a soluble metasilicate and/or metagermanate, optionally in combination with a carboxylate, and (ii) an aqueous solution comprising a metal salt.

7. Method according to any one of the previous claims, wherein the said noble metal source is an aqueous solution comprising a soluble noble metal salt.

8. Method according to claim 6 or 7, wherein the said soluble metasilicate and/or the said soluble metagermanate in step (a) are selected from the respective sodium or potassium salts, or mixtures thereof, and/or wherein the said optional carboxylate in step (a) is a metal carboxylate, the metal being selected from sodium or potassium, or mixtures thereof.

9. Method according to any one of claims 6 to 8, wherein the said optional carboxylate in step (a) is selected from formate, acetate, propionate, butyrate, isobutyrate, valerate, isovalerate or a mixture thereof, and/or wherein the metal in the said soluble metal salt in step (a) is a divalent metal selected from magnesium, cobalt, zinc, copper, manganese, iron, nickel, chromium or mixtures thereof, for example wherein the said soluble metal salt is magnesium acetate.

10. Method according to any one of claims 7 to 9, wherein the noble metal in the said soluble noble metal salt in step (b) is selected from gold, palladium, platinum, iridium, ruthenium, rhodium, or mixtures thereof.

11. Method according to any one of claims 1 to 10, wherein the said combination step (c) is carried out by mixing the said noble metal source of step (b) with the said component (i) of the phyllomineral source of step (a) and the said component (ii) of the phyllomineral source of step (a)in any order.

12. Method according to any one of claims 1 to 11, wherein the said hydrothermal treatment of step (d) is carried out by heating at a temperature between 100°C and 600°C for a duration of 10 minutes to 30 days, and wherein the heating is achieved by microwave heating, by convection heating, or combinations thereof.

13. Method according to any one of claims 1 to 12, wherein the product obtained after the combination step (c) or after hydrothermal treatment step (d) is dried, washed and/or centrifiged.

14. Method according to any one of the previous claims, which carried out as a batch process in an autoclave under autogeneous pressure.

15. Method according to any one of claims 1 to 13, which carried out as a continuous process under a pressure of 1 MPa or higher.

16. Particulate composition comprising a combination of phyllomineral with noble metal obtained according to any one of the previous claims.

17. Particulate composition according to claim 16, wherein the phyllomineral is a natural or synthetic phyllomineral selected from talc, mica, chlorite, kaolinite, or mixtures thereof, and/or wherein the said noble metal is selected from the group consisting of gold, palladium, platinum, iridium, ruthenium, rhodium, or mixtures thereof.

18. Particulate composition according to claim 16 or 17, wherein the said noble metal is in the form of nanoparticles intimately linked to the phyllomineral surface.

19. Particulate composition according to claim 18, wherein the said noble metal nanoparticles have an average particle diameter in the range of 1 nm to 100 nm, and/or wherein the said phyllomineral particles are platelets having a large diameter in the range of 10 nm to 1 mm.

20. Use of the particulate composition of any one of claims 16 to 19, or of the method of any one of claims 1 to 15, in the production of catalysts, cosmetics, pharmaceuticals or fillers.
